# EUROPEAN PATENT APPLICATION

(11) **EP 4 506 336 A1**
(43) Date of publication of application: **12.02.2025**
(21) Application number: 23780884.5
(22) Date of filing: 30.03.2023
(51) Int. Cl.: C07C 319/12, C07C 321/04, C07B 61/00, C08G 18/38, C08G 18/73, C08G 18/75, C08G 18/76, G02B 1/04

(54) **METHOD FOR PRODUCING PENTAERYTHRITOL MERCAPTOCARBOXYLIC ESTER, POLYMERIZABLE COMPOSITION, RESIN, OPTICAL MATERIAL, AND SPECTACLE LENS**

(30) Priority: 30.03.2022 JP 2022056070
(71) Applicant: HOYA LENS THAILAND LTD., Pathumthani 12130 (TH)
(72) Inventor: KOUSAKA, Masahisa, Tokyo 160-8347 (JP)
(74) Representative: Kraus & Lederer PartGmbB
(86) International application number: PCT/JP2023/013189
(87) International publication number: WO 2023/190876

(57) **Abstract**

There are provided a method of producing pentaerythritol mercaptocarboxylate including subjecting pentaerythritol and mercaptocarboxylic acid to a dehydration reaction, wherein the pentaerythritol has an absorbance of 0.1 or more at a wavelength of 270 nm in an aqueous solution containing 5 mass% of the pentaerythritol measured in a quartz cell with an optical path length of 50 mm, a polymerizable composition containing the pentaerythritol mercaptocarboxylate, a resin which is a cured product of the polymerizable composition, an optical material containing the resin, and a spectacle lens containing the resin.

## Description

### [Technical Field]

The present disclosure relates to a method of producing pentaerythritol mercaptocarboxylate, a polymerizable composition, a resin, an optical material, and a spectacle lens.

### [Background Art]

Plastic spectacle lenses are widely known in the related art. Polythiourethane resins are used as a material for spectacle lenses (for example, PTL 1). Since the polythiourethane resin is a material having an appropriate refractive index, it allows a spectacle lens to have an appropriate thickness, and easily secures processability and impact resistance, and is widely used.

Pentaerythritol mercaptocarboxylate is used as a monomer for a polythiourethane resin. As a method of synthesizing the compound, PTL 2 describes a method of producing pentaerythritol mercaptocarboxylate including reacting pentaerythritol with a mercaptocarboxylic acid, and regarding the pentaerythritol, an aqueous solution containing 5 weight% of pentaerythritol is prepared, and one having an absorbance of 0.07 or less at a wavelength of 270 nm in an aqueous solution measured in a quartz cell with an optical path length of 50 mm is selected. It is described that, according to the production method, it is possible to stably produce a colorless and transparent resin molded component that is excellent in appearance such as hue.

### [Citation List]

### [Patent Literature]

[PTL 1] Japanese Patent Application Publication No. 2004-002820
[PTL 2] WO 2016/208707

### [Summary of Invention]

### [Technical Problem]

One embodiment of the present disclosure provides a method of producing pentaerythritol mercaptocarboxylate which allows a resin with little coloration and little cloudiness to be obtained, a polymerizable composition containing the pentaerythritol mercaptocarboxylate, a resin which is a cured product of the polymerizable composition, an optical material containing the resin, and a spectacle lens containing the resin.

### [Solution to Problem]

The inventors surprisingly found that the above objects can be achieved by the following configuration, which is the exact opposite to the solution described in the above PTL 2. That is, the inventors found that, when pentaerythritol having an absorbance of 0.1 or more at a wavelength of 270 nm in a 5 mass% aqueous solution measured in a quartz cell with an optical path length of 50 mm is used, pentaerythritol mercaptocarboxylate which allows a resin with little coloration and little cloudiness to be obtained is obtained.

One embodiment according to the present disclosure relates to a method of producing pentaerythritol mercaptocarboxylate including subjecting pentaerythritol and a mercaptocarboxylic acid to a dehydration reaction, wherein the pentaerythritol has an absorbance of 0.1 or more at a wavelength of 270 nm in an aqueous solution containing 5 mass% of the pentaerythritol measured in a quartz cell with an optical path length of 50 mm.

### [Advantageous Effects of Invention]

According to one embodiment of the present disclosure, it is possible to provide a method of producing pentaerythritol mercaptocarboxylate which allows a resin with little coloration and little cloudiness to be obtained, a polymerizable composition containing the pentaerythritol mercaptocarboxylate, a resin which is a cured product of the polymerizable composition, an optical material containing the resin, and a spectacle lens containing the resin.

### [Description of Embodiments]

Hereinafter, an embodiment (hereinafter referred to as "the present embodiment") of the present disclosure will be described in detail, but the present invention is not limited thereto, and can be variously modified without departing from the scope and spirit thereof. Here, in this specification, for example, the expression of a numerical value range of "1 to 100" includes both the lower limit value "1" and the upper limit value "100." In addition, the same applies to the expression of other numerical value ranges.

### [Method of producing pentaerythritol mercaptocarboxylate]

One embodiment according to the present disclosure relates to a method of producing pentaerythritol mercaptocarboxylate including subjecting pentaerythritol and mercaptocarboxylic acid to a dehydration reaction, wherein the pentaerythritol has an absorbance of 0.1 or more at a wavelength of 270 nm in an aqueous solution containing 5 mass% of the pentaerythritol measured in a quartz cell with an optical path length of 50 mm. According to one embodiment of the present disclosure, it is possible to provide a method of producing pentaerythritol mercaptocarboxylate which allows a resin with little coloration and little cloudiness to be obtained, a polymerizable composition containing the pentaerythritol mercaptocarboxylate, a resin which is a cured product of the polymerizable composition, an optical material containing the resin, and a spectacle lens containing the resin.

### <Absorbance in aqueous solution containing 5 mass% of pentaerythritol at wavelength of 270 nm>

The pentaerythritol used in the present embodiment has an absorbance of 0.1 or of more at a wavelength of 270 nm in an aqueous solution containing 5 mass% of pentaerythritol measured in a quartz cell with an optical path length of 50 mm. It is surprisingly found that, even when pentaerythritol exhibiting an absorbance in this range is used, a resin with little coloration and little cloudiness can be obtained. The absorbance is preferably 0.3 or more, more preferably 0.5 or more, and still more preferably 1.0 or more. The upper limit of the absorbance is not particularly limited, and may be 20 or less, is preferably 10 or less, more preferably 5.0 or less, still more preferably 3.0 or less, and yet more preferably 2.0 or less. The method of measuring the absorbance is the method described in examples. In order to set the absorbance to be within the range, for example, pentaerythritol can be purified to adjust the absorbance to be within the range.

A method of producing pentaerythritol mercaptocarboxylate according to the present embodiment includes, for example,
subjecting a mercaptocarboxylic acid and pentaerythritol to a dehydration reaction (hereinafter referred to as a "reaction process"), and
washing the pentaerythritol mercaptocarboxylate obtained by the dehydration reaction (hereinafter referred to as a "washing process").

The processes will be described below in detail.

### [Reaction process]

In the reaction process, a mercaptocarboxylic acid and pentaerythritol are subjected to a dehydration reaction under solvent-free conditions. In the reaction process, it is preferable to perform the reaction under solvent-free conditions. This makes it unnecessary to distill off the solvent, and thus it is possible to reduce the load of subsequent purification.

Here, "under solvent-free conditions" means conditions in which no organic solvent such as toluene is used, and specifically means conditions in which the amount of the organic solvent is 20 mass% or less based on a total mass of mercaptocarboxylic acid and pentaerythritol. The amount of the organic solvent is preferably 10 mass% or less, more preferably 5 mass% or less, and still more preferably 1 mass% or less based on a total mass of mercaptocarboxylic acid and pentaerythritol.

### (Mercaptocarboxylic acid)

Mercaptocarboxylic acid preferably has 2 to 4 carbon atoms and more preferably has 2 to 3 carbon atoms. Mercaptocarboxylic acid is preferably 2-mercaptoacetic acid or 3-mercaptopropionic acid. In addition, an ester bond is formed by a dehydration reaction which is a reaction between mercaptocarboxylic acid and pentaerythritol, and pentaerythritol mercaptocarboxylate is obtained.

The pentaerythritol preferably has a purity of 90 mass% or more, more preferably a purity of 95 mass% or more, and still more preferably has a purity of 98 mass% or more.

The pentaerythritol may contain more than 1.0 mass% of sodium. Even when it contains such a high concentration of sodium, pentaerythritol mercaptocarboxylate having a low Hazen color scale value (APHA) is obtained.

The molar ratio between mercaptocarboxylic acid and pentaerythritol (mercaptocarboxylic acid/pentaerythritol) is preferably 3.5 to 6.0, more preferably 4.0 to 5.0, and still more preferably 4.1 to 5.5.

In the reaction process, the dehydration condensation is preferably performed in the presence of a sulfonic acid compound. The sulfonic acid compound acts as an acid catalyst and promotes the dehydration reaction.

Examples of sulfonic acid compounds include aromatic sulfonic acids such as toluenesulfonic acid, and alkylsulfonic acids. Among these, alkylsulfonic acids are preferable in order to reduce coloration. Examples of alkylsulfonic acids include methanesulfonic acid, ethanesulfonic acid, 1-propanesulfonic acid, and trifluoromethanesulfonic acid. Among these, methanesulfonic acid is preferable in order to reduce coloration.

The amount of the sulfonic acid compound is preferably 0.1 to 10 mass%, more preferably 0.2 to 5 mass%, and still more preferably 0.3 to 3 mass% based on a total amount of mercaptocarboxylic acid and pentaerythritol.

The reaction process is preferably performed under a reduced pressure while removing water from the system. When water is removed from the system, the equilibrium of the ester reaction can be shifted toward the product and the yield can be improved.

The reaction process is preferably performed at a temperature of 90 to 98°C in order to reduce coloration. The temperature during the reaction process is preferably 92 to 97°C and more preferably 93 to 96°C.

### [Washing process]

In the washing process, the pentaerythritol mercaptocarboxylate obtained in the reaction process is washed. The washing is performed, for example, by washing the mixture obtained in the reaction process with water. Thereby, the catalyst such as a sulfonic acid compound can be removed.

### [Pentaerythritol mercaptocarboxylate]

Pentaerythritol mercaptocarboxylate is obtained by the production method according to the present embodiment.

The Hazen color scale value (APHA) of the obtained pentaerythritol mercaptocarboxylate is preferably 15 or less, more preferably 10 or less, still more preferably 7 or less, and yet more preferably 6 or less.

The measured value (g/eq) of the SH value of the obtained pentaerythritol mercaptocarboxylate/theoretical value (g/eq) of the SH value of the pentaerythritol mercaptocarboxylate (measured value/theoretical value) is preferably 1.10 or less, more preferably 1.08 or less, and still more preferably 1.06 or less. The measured value of the SH value is obtained by the measurement method described in examples. The theoretical value of the SH value is obtained by the following Formula (A). [theoretical value of SH value]=[molecular weight]/[number of SH groups in one molecule]

### [Polymerizable composition]

The polymerizable composition according to the present embodiment contains the pentaerythritol mercaptocarboxylate obtained by the production method according to the present embodiment and a polyiso(thio)cyanate compound. The pentaerythritol mercaptocarboxylate is polymerized with reacting with the polyiso(thio)cyanate to obtain a cured product.

### (Polyiso(thio)cyanate)

Examples of polyiso(thio)cyanates include polyisocyanate compounds having an aromatic ring, alicyclic polyisocyanate compounds, and linear or branched aliphatic polyisocyanate compounds.

Examples of polyisocyanate compounds having an aromatic ring include diisocyanatobenzene, 2,4-tolylene diisocyanate, 2,6-tolylene diisocyanate, ethyl phenylene diisocyanate, isopropyl phenylene diisocyanate, dimethyl phenylene diisocyanate, diethyl phenylene diisocyanate, diisopropyl phenylene diisocyanate, trimethylbenzene triisocyanate, benzene triisocyanate, biphenyl diisocyanate, toluidine diisocyanate, 4,4'-diphenylmethane diisocyanate, 4,4'-methylenebis(2-methylphenylisocyanate), bibenzyl-4,4'-diisocyanate, bis(isocyanatophenyl)ethylene, 1,3-bis(isocyanatomethyl)benzene, 1,4-bis(isocyanatomethyl)benzene, 1,3-bis(isocyanatoethyl)benzene, bis(isocyanatopropyl)benzene, α,α,α',α'-tetramethylxylylene diisocyanate, bis(isocyanatobutyl)benzene, bis(isocyanatomethyl)naphthalene, bis(isocyanatomethylphenyl)ether, 2-isocyanatophenyl-4-isocyanatophenyl sulfide, bis(4-isocyanatophenyl)sulfide, bis(4-isocyanatomethylphenyl)sulfide, bis(4-isocyanatophenyl)disulfide, bis(2-methyl-5-isocyanatophenyl)disulfide, bis(3-methyl-5-isocyanatophenyl)disulfide, bis(3-methyl-6-isocyanatophenyl)disulfide, bis(4-methyl-5-isocyanatophenyl)disulfide, bis(3-methyloxy-4-isocyanatophenyl)disulfide, and bis(4-methyloxy-3-isocyanatophenyl)disulfide.

Examples of alicyclic polyisocyanate compounds include 1,3-diisocyanatocyclohexane, isophorone diisocyanate, 1,3-bis(isocyanatomethyl)cyclohexane, 1,4-bis(isocyanatomethyl)cyclohexane, dicyclohexylmethane-4,4'-diisocyanate, 2,5-bis(isocyanatomethyl)-bicyclo[2.2.1]heptane, 2,6-bis(isocyanatomethyl)-bicyclo[2.2.1]heptane, 2,5-diisocyanato-1,4-dithiane, 2,5-bis(isocyanatomethyl)-1,4-dithiane, 4,5-diisocyanato-1,3-dithiolane, 4,5-bis(isocyanatomethyl)-1,3-dithiolane, and 4,5-bis(isocyanatomethyl)-2-methyl-1,3-dithiolane.

Examples of linear or branched aliphatic polyisocyanate compounds include hexamethylene diisocyanate, 2,2-dimethylpentane diisocyanate, 2,2,4-trimethylhexane diisocyanate, butene diisocyanate, 1,3-butadiene-1,4-diisocyanate, 2,4,4-trimethylhexamethylene diisocyanate, 1,6,11-undecane triisocyanate, 1,3,6-hexamethylenetriisocyanate, 1,8-diisocyanato-4-isocyanatomethyloctane, bis(isocyanatoethyl)carbonate, bis(isocyanatoethyl)ether, lysine diisocyanatomethyl ester, lysine triisocyanate, bis(isocyanatomethyl)sulfide, bis(isocyanatoethyl)sulfide, bis(isocyanatopropyl)sulfide, bis(isocyanatohexyl)sulfide, bis(isocyanatomethyl)sulfone, bis(isocyanatomethyl)disulfide, bis(isocyanatoethyl)disulfide, bis(isocyanatopropyl)disulfide, bis(isocyanatomethylthio)methane, bis(isocyanatoethylthio)methane, bis(isocyanatomethylthio)ethane, bis(isocyanatoethylthio)ethane, 1,5-diisocyanato-2-isocyanatomethyl-3-pentane, 1,2,3-tris(isocyanatomethylthio)propane, 1,2,3-tris(isocyanatoethylthio)propane, 3,5-dithia-1,2,6,7-heptanetetraisocyanate, 2,6-diisocyanatomethyl-3,5-dithia-1,7-heptane diisocyanate, 2,5-diisocyanatomethylthiophene, 4-isocyanatoethylthio-2,6-dithia-1,8-octanediisocyanate, 1,2-diisothiocyanatoethane, and 1,6-diisothiocyanatohexane.

These may be used alone or two or more thereof may be used.

The polyiso(thio)cyanate preferably contains at least one selected from the group consisting of bis(isocyanatomethyl)bicyclo[2.2.1]heptane, bis(isocyanatomethyl)cyclohexane, bis(isocyanatomethyl)benzene, tolylene diisocyanate, diphenylmethane diisocyanate, dicyclohexylmethane diisocyanate, hexamethylene diisocyanate, and pentamethylene diisocyanate (hereinafter referred to as "preferable isocyanate compound"), and more preferably contains at least one selected from the group consisting of bis(isocyanatomethyl)benzene, tolylene diisocyanate, and diphenylmethane diisocyanate.

Bis(isocyanatomethyl)bicyclo[2.2.1]heptane includes, for example, one or more selected from the group consisting of 2,5-bis(isocyanatomethyl)bicyclo[2.2.1]heptane and 2,6-bis(isocyanatomethyl)bicyclo[2.2.1]heptane, and is preferably a mixture of 2,5-bis(isocyanatomethyl)bicyclo[2.2.1]heptane and 2,6-bis(isocyanatomethyl)bicyclo[2.2.1]heptane.

Examples of bis(isocyanatomethyl)cyclohexane include 1,3-bis(isocyanatomethyl)cyclohexane and 1,4-bis(isocyanatomethyl)cyclohexane. Among these, 1,3-bis(isocyanatomethyl)cyclohexane is preferable.

Examples of bis(isocyanatomethyl)benzene include 1,3-bis(isocyanatomethyl)benzene and 1,4-bis(isocyanatomethyl)benzene. Among these, 1,3-bis(isocyanatomethyl)benzene is preferable.

Examples of tolylene diisocyanates include 2,4-tolylene diisocyanate and 2,6-tolylene diisocyanate. Among these, 2,4-tolylene diisocyanate is preferable.

Examples of diphenylmethane diisocyanates include 4,4'-diphenylmethane diisocyanate.

Examples of dicyclohexylmethane diisocyanates include dicyclohexylmethane-4,4'-diisocyanate.

The polyiso(thio)cyanate compound preferably includes at least one selected from among bis(isocyanatomethyl)-bicyclo[2.2.1]heptane, bis(isocyanatomethyl)cyclohexane, bis(isocyanatomethyl)benzene, isophorone diisocyanate, pentamethylene diisocyanate, and hexamethylene diisocyanate, and among these, bis(isocyanatomethyl)bicyclo[2.2.1]heptane, bis(isocyanatomethyl)cyclohexane, or bis(isocyanatomethyl)benzene is preferable.

In the polyiso(thio)cyanate, the content of the above "preferable isocyanate compound" is preferably 80 mass% or more, more preferably 90 mass% or more, and still more preferably 95 mass% or more and 100 mass% or less.

The equivalent ratio between the mercapto groups of the pentaerythritol mercaptocarboxylate and the isocyanate groups of the polyiso(thio)cyanate (mercapto groups/isocyanate groups) is preferably 40/60 or more, more preferably 43/57 or more, still more preferably 45/55 or more, and preferably 60/40 or less, more preferably 55/45 or less, and still more preferably 53/47 or less.

A polymerization catalyst may be used for curing the isocyanate component and the thiol component.

Examples of polymerization catalysts include tin compounds and nitrogen-containing compounds.

Examples of tin compounds include alkyltin compounds and alkyltin halide compounds.

Examples of alkyltin compounds include dibutyltin diacetate and dibutyltin dilaurate.

Examples of alkyltin halide compounds include dibutyltin dichloride, dimethyltin dichloride, monomethyltin trichloride, trimethyltin chloride, tributyltin chloride, tributyltin fluoride, and dimethyltin dibromide.

Among these, dibutyltin diacetate, dibutyltin dilaurate, dibutyltin dichloride, and dimethyltin dichloride are preferable, and dimethyltin dichloride is more preferable.

Examples of nitrogen-containing compounds include tertiary amines, quaternary ammonium salts, imidazole compounds, and pyrazole compounds. Tertiary amines are preferably hindered amines.

Examples of tertiary amines include triethylamine, tri-n-propylamine, triisopropylamine, tri-n-butylamine, triisobutylamine, N,N-dimethylbenzylamine, N-methylmorpholine, N,N-dimethylcyclohexylamine, pentamethyldiethylenetriamine, bis(2-dimethylaminoethyl) ether, N-methylmorpholine, N,N'-dimethylpiperazine, N,N,N',N'-tetramethylethylenediamine, and 1,4-diazabicyclo[2.2.2]octane(DABCO).

Examples of hindered amines include 1,2,2,6,6-pentamethyl-4-piperidinol, 1,2,2,6,6-pentamethyl-4-hydroxyethyl-4-piperidinol, methyl-1,2,2,6,6-pentamethyl-4-piperidyl sebacate, a mixture of methyl-1,2,2,6,6-pentamethyl-4-piperidyl sebacate and bis(1,2,2,6,6-pentamethyl-4-piperidyl)sebacate, bis(1,2,2,6,6-pentamethyl-4-piperidyl)sebacate, bis(2,2,6,6-tetramethyl-1-(octyloxy)-4-piperidyl)sebacate, bis(1,2,2,6,6-pentamethyl-4-piperidyl) [[3,5-bis(1,1-dimethylethyl)-4-hydroxyphenyl]methyl]butylmalonate, and tetrakis(1,2,2,6,6-pentamethyl-4-piperidyl)butane-1,2,3,4-tetracarboxylate.

Examples of quaternary ammonium salts include tetraethylammonium hydroxide.

Examples of imidazole compounds include imidazole, 1-methyl-2-mercapto-1H-imidazole, 1,2-dimethylimidazole, benzylmethylimidazole, and 2-ethyl-4-imidazole.

Examples of pyrazole compounds include pyrazole and 3,5-dimethylpyrazole.

Among these, tertiary amines such as hindered amines, imidazole compounds, and pyrazole compounds are preferable, imidazole compounds are more preferable, and 1-methyl-2-mercapto-1H-imidazole is still more preferable.

The amount of the polymerization catalyst used is preferably 0.001 to 2 parts by mass, more preferably 0.005 to 1 part by mass, and still more preferably 0.007 to 0.5 parts by mass based on a total amount of 100 parts by mass of the isocyanate component and the thiol component.

### [Resin]

The resin according to the present embodiment is a cured product of the polymerizable composition according to the above embodiment. The cured product is obtained by polymerizing the components in the polymerizable composition.

Polymerization conditions can be appropriately set depending on the polymerizable composition. The polymerization start temperature is preferably 0°C or higher, more preferably 10°C or higher, and preferably 50°C or lower, and more preferably 40°C or lower. Preferably, the temperature is raised from the polymerization start temperature and curing by heating is then performed. For example, the maximum heating temperature is generally 110°C or higher and 130°C or lower.

Resins are used in various optical materials such as spectacle lenses, camera lenses, prisms, optical fibers, substrates used therefor, substrates for recording media used for optical disks, magnetic disks or the like, and optical filters attached to computer displays. Among these optical materials, resins are preferably used as spectacle lenses.

### [Spectacle lens]

The spectacle lens according to the present embodiment contains the resin according to the present embodiment.

In addition, the spectacle lens according to the present embodiment preferably includes a lens substrate containing the resin according to the present embodiment.

### (Lens substrate)

The lens substrate contains the resin according to the present embodiment in an amount of preferably 90 mass% or more, more preferably 95 mass% or more, and still more preferably 99 mass% or more.

The lens substrate may contain other additives such as a mold releasing agent, a coloring agent, an antioxidant, an anti-coloring agent, and a fluorescent brightening agent. These may be used alone or two or more thereof may be used.

The lens substrate may be either a finished lens or a semi-finished lens. The surface shape of the lens substrate is not particularly limited, and may be flat, convex, concave or the like. The lens substrate may be used for any application such as a single focal lens, a multifocal lens, and a progressive power lens. For example, as an example, for a progressive power lens, generally, a near portion region (near portion) and a progressive portion region (intermediate region) are included in the above lower region, and a distance portion region (distance portion) is included in an upper region. As the lens substrate, a colorless lens substrate is generally used, but a lens substrate that is colored in a range in which the transparency is not impaired can also be used.

The refractive index ne of the lens substrate is preferably 1.60 or more. The upper limit of the refractive index ne of the lens substrate is not particularly limited, and may be, for example, 1.80 or less.

The spectacle lens according to the present embodiment may include at least one layer selected from the group consisting of a hard coat layer, a foundation layer, and an antireflection layer.

### [Method of producing spectacle lens]

The method of producing a spectacle lens according to the present embodiment includes curing, in a molding die, a polymerizable composition containing a thiol component including the compound (1) obtained by the production method of the present embodiment and an isocyanate component.

The amount of odor is reduced using the compound obtained by the production method of the compound (1) according to the above embodiment. In addition, a polythiourethane resin having a high refractive index and a colorless and transparent appearance is obtained by the method of producing a spectacle lens according to the present embodiment.

The method of producing a spectacle lens according to the present embodiment may include annealing the cured resin.

The polymerization is preferably a cast polymerization method. For example, the lens substrate can be obtained by injecting a polymerizable composition into a mold in which a glass or metal mold and a tape or a gasket are combined and performing polymerization.

Polymerization conditions can be appropriately set depending on the polymerizable composition. The polymerization start temperature is preferably 0°C or higher, more preferably 10°C or higher, and preferably 50°C or lower, and more preferably 40°C or lower. Preferably, the temperature is raised from the polymerization start temperature and curing by heating is then performed. For example, the maximum heating temperature is generally 110°C or higher and 130°C or lower.

After the polymerization is completed, the lens substrate may be released, and the annealing treatment may be performed. The temperature during the annealing treatment is preferably 100 to 150°C.

### [Examples]

Hereinafter, the present embodiment will be described in more detail with reference to examples and comparative examples. Here, the present invention is not limited to the following examples.

### [Measurement method]

### <Absorbance in aqueous solution containing 5 mass% of pentaerythritol at wavelength of 270 nm>

Distilled water was added to 5 parts by mass of pentaerythritol to prepare 100 parts by mass of a sample solution. Next, the sample solution was heated to 60°C and stirred for 30 minutes. The sample solution was cooled to 20°C and filtered through a 0.45 µm filter to obtain an aqueous solution, which was used as a measurement sample. 1 hour after filtering was completed at room temperature (25°C), the measurement sample solution was put into a quartz cell with an optical path length of 50 mm, and the absorbance at a wavelength of 270 nm was obtained using a spectrophotometer (device name: UH4150, commercially available from Hitachi High-Tech Corporation).

### <Hazen color scale value (APHA)>

The obtained monomer was put into a quartz cell with an optical path length of 50 mm, and the Hazen color scale value (APHA) was measured by the method described in JIS K0071-1: 2017.

### <Opacity>

The degree of opacity of the resin obtained by thermal polymerization of the polymerizable composition was evaluated on the basis of the following criteria.

### (Evaluation criteria)

A: No opacity was observed in the resin cured by thermal polymerization
B: Opacity was observed in the resin cured by thermal polymerization, but the resin was transparent with scattered opaque dots
C: Opacity was observed in the resin cured by thermal polymerization, the entire resin was opaque, and the entire resin was white.

### <Example 1>

74.6 parts by mass (0.548 mol) of pentaerythritol, which had an absorbance of 1.40 measured by the above method, 240.0 parts by mass (2.261 mol) of 3-mercaptopropionic acid and 1.67 parts by mass of methanesulfonic acid were put into a 1,000 mL flask, and the flask was immersed in an oil bath whose temperature was controlled to about 100°C and stirring and reaction were performed while degassing using a vacuum pump for 3 hours (an internal temperature of 97°C).

After the reaction was completed, the sample was washed with 400 mL of water three times, and water was removed using a vacuum pump. The refractive index n_{d}(25°C) of the synthesized pentaerythritol mercaptocarboxylate was 1.528, the SHV was 125.62, and the APHA value was 5.

53.15 parts by mass of 1,3-bis(isocyanatomethyl)cyclohexane (HXDI), 0.22 parts by mass of acidic phosphate ester "JP506H" (product name, commercially available from Johoku Chemical Co., Ltd.), 0.072 parts by mass of dimethyltin dichloride (DMTDCl), and 0.12 parts by mass of a UV absorbing agent "SEESORB707" (product name, commercially available from Shipro Kasei Kaisha, Ltd.) were mixed and dissolved, 66.85 parts by mass of the pentaerythritol mercaptocarboxylate described above was added, the mixture was stirred and degassed for 30 minutes to make it homogeneous, the mixture was filtered through a 10-micron PTFE filter, and 50 g of the mixture was poured into a glass sample bottle and sealed, and additionally injected into a flat glass mold adjusted to a thickness of 3 mm. The mixture was polymerized by heating from 20°C to 120°C for 24 hours and cooled to near room temperature, and the resin cylinder in the sample bottle and the 3 cm-thick flat plate were removed. The obtained resin cylinder and flat plate were evaluated as described above.

### [Table 1]

**Table 1**

| | | Example 1 |
|---|---|---|
| Pentaerythritol | Absorbance | 1.40 |
| | Amount (g) | 74.6 |
| 3-mercaptopropionic acid | Amount (g) | 240.0 |
| Methanesulfonic acid | Amount (g) | 1.67 |
| Pentaerythritol mercaptocarboxylate evaluation | Refractive index (25°C) | 1.53 |
| | SHV | 125.6 |
| | APHA | 5 |
| Evaluation after polymerization | Opacity | A |

On the basis of the above results, it was surprisingly found that, when pentaerythritol having an absorbance of 0.1 or more at a wavelength of 270 nm in a 5 mass% aqueous solution measured in a quartz cell with an optical path length of 50 mm was used, pentaerythritol mercaptocarboxylate which allowed a resin with little coloration and little cloudiness to be obtained was obtained.

## Claims

1. A method of producing pentaerythritol mercaptocarboxylate, comprising
subjecting pentaerythritol and mercaptocarboxylic acid to a dehydration reaction,
wherein the pentaerythritol has an absorbance of 0.1 or more at a wavelength of 270 nm in an aqueous solution containing 5 mass% of the pentaerythritol measured in a quartz cell with an optical path length of 50 mm.

2. The method of producing pentaerythritol mercaptocarboxylate according to claim 1,
wherein the mercaptocarboxylic acid has 2 to 4 carbon atoms.

3. The method of producing pentaerythritol mercaptocarboxylate according to claim 1,
wherein the mercaptocarboxylic acid is mercaptoacetic acid or mercaptopropionic acid.

4. The method of producing pentaerythritol mercaptocarboxylate according to claim 1,
wherein the dehydration condensation is performed in the presence of a sulfonic acid compound.

5. The method of producing pentaerythritol mercaptocarboxylate according to claim 4,
wherein the amount of the sulfonic acid compound is 0.1 to 10 mass% based on a total amount of the mercaptocarboxylic acid and the pentaerythritol.

6. The method of producing pentaerythritol mercaptocarboxylate according to claim 4,
wherein the sulfonic acid compound is an alkylsulfonic acid.

7. The method of producing pentaerythritol mercaptocarboxylate according to claim 1,
wherein the dehydration reaction is performed under a reduced pressure while removing water from the system.

8. The method of producing pentaerythritol mercaptocarboxylate according to claim 1,
wherein the dehydration reaction is performed at a temperature of 90 to 98°C.

9. The method of producing pentaerythritol mercaptocarboxylate according to claim 1,
wherein the pentaerythritol mercaptocarboxylate has an APHA value of 15 or less.

10. The method of producing pentaerythritol mercaptocarboxylate according to claim 1, comprising
washing the pentaerythritol mercaptocarboxylate obtained by the dehydration reaction.

11. A polymerizable composition comprising the pentaerythritol mercaptocarboxylate obtained by the production method according to any one of claims 1 to 10 and a polyiso(thio)cyanate.

12. The polymerizable composition according to claim 11,
wherein the polyiso(thio)cyanate includes at least one selected from among bis(isocyanatomethyl)-bicyclo[2.2.1]heptane, bis(isocyanatomethyl)cyclohexane, bis(isocyanatomethyl)benzene, isophorone diisocyanate, pentamethylene diisocyanate, and hexamethylene diisocyanate.

13. A resin which is a cured product of the polymerizable composition according to claim 11.

14. An optical material comprising the resin according to claim 13.

15. A spectacle lens comprising the resin according to claim 14.
